# EUROPÄISCHE PATENTSCHRIFT

(11) **EP 1 189 861 B1**
(45) Veröffentlichungstag und Bekanntmachung des Hinweises auf die Patenterteilung: **10.12.2003**
(21) Anmeldenummer: 00947834.8
(22) Anmeldetag: 31.05.2000
(51) Int. Cl.: C07C 57/07, C07C 57/05, C07C 51/43

(54) **VERFAHREN ZUR REININGUNG UND HERSTELLUNG VON ACRYLSÄURE ODER METHACRYLSÄURE**
METHOD OF PURIFYING AND PRODUCING ACRYLIC ACID OR METHACRYLIC ACID
PROCEDE DE PURIFICATION ET DE PREPARATION D'ACIDE ACRYLIQUE OU METHACRYLIQUE

(30) Priorität: 08.06.1999 DE 19926082
(43) Veröffentlichungstag der Anmeldung: 27.03.2002
(73) Patentinhaber: BASF AKTIENGESELLSCHAFT, 67056 Ludwigshafen (DE)
(72) Erfinder: ECK, Bernd, D-68519 Viernheim (DE); HEILEK, Jörg, D-69245 Bammental (DE); BAUMANN, Dieter, D-69190 Walldorf (DE); SCHLIEPHAKE, Volker, D-67105 Schifferstadt (DE)
(74) Vertreter: Isenbruck, Günter, Dr.
(86) Internationale Anmeldenummer: EP0005004
(87) Internationale Veröffentlichungsnummer: WO00075097

(56) Entgegenhaltungen:
- EP-A- 0 974 574
- DE-A- 19 627 847
- DE-A- 19 740 252
- DE-A- 19 833 049

## Beschreibung

Die vorliegende Erfindung betrifft ein Verfahren zur Reinigung von Acrylsäure oder Methacrylsäure. Außerdem betrifft die Erfindung ein Verfahren zur Herstellung von Acrylsäure oder Methacrylsäure unter Verwendung des Reinigungsverfahrens.

Acrylsäure und Methacrylsäure sind bedeutende Grundchemikalien. Aufgrund ihrer sehr reaktionsfähigen Doppelbindung sowie der Säurefunktion eignet sich insbesondere die Acrylsäure als Monomeres zur Herstellung von Polymerisaten, z. B. für Klebstoffe, Dispersionen, Lacke oder "Superabsorber".

Aus WO-A-9 801 415 ist ein Verfahren zur Herstellung von Acrylsäure oder Methacrylsäure bekannt, bei dem zuerst ein die Säure enthaltendes Produktgemisch hergestellt wird, dieses kondensiert wird und anschließend die Säure aus der bei der Kondensation erhaltenen Lösung kristallisiert wird.

Die Aufgabe der vorliegenden Erfindung bestand darin, die Reinheit von Acrylsäure oder Methacrylsäure zu erhöhen.

Zur Lösung dieser Aufgabe wird vorgeschlagen, daß ein Kristallisat von Acrylsäure oder Methacrylsäure mit einer die entsprechende Säure enthaltenden Wasch- oder Reinigungsflüssigkeit mit einer Temperatur von 15 bis 40°C gewaschen wird. Überraschenderweise wurde festgestellt, daß mit dieser Maßnahme auf sehr einfache Art und Weise eine sehr gute Reinigungswirkung erzielt wird.

Somit betrifft die Erfindung ein Verfahren zur Reinigung von Acrylsäure oder Methacrylsäure durch Kristallisation unter Erhalt eines Kristallisats und einer Mutterlauge, das dadurch gekennzeichnet ist, daß das Kristallisat mit einer Acrylsäure oder Methacrylsäure enthaltenden Waschflüssigkeit mit einer Temperatur von 15 bis 40°C gewaschen wird.

In einer Ausgestaltung betrifft die Erfindung ein Verfahren zur Herstellung von Acrylsäure oder Methacrylsäure, das folgende Stufen umfaßt:
(a) Herstellung eines gasförmigen Produktgemisches, das im wesentlichen die Zusammensetzung eines Reaktionsgemisches der katalytischen Gasphasenoxidation von C₃- bzw. C₄-Alkanen, -Alkenen, -Alkanolen und/oder -Alkanalen und/oder Vorstufen davon zu Acrylsäure oder Methacrylsäure hat,
(b) Kondensation des gasförmigen Produktgemisches,
(c) Kristallisation der Acrylsäure oder Methacrylsäure aus der in Stufe (b) erhaltenen Lösung und
(d) Reinigung der Acrylsäure bzw. Methacrylsäure aus dem Kristallisat der vorhergehenden Stufe gemäß dem oben beschriebenen erfindungsgemäßen Reinigungsverfahren.

Bevorzugte Ausführungsformen der Erfindung sind in der nachfolgenden Beschreibung, dem Beispiel und den Unteransprüchen definiert.

Erfindungsgemäß wird das Acrylsäure- bzw. Methacrylsäurekristallisat mit einer die entsprechende Säure enthaltenden Waschflüssigkeit oder Reinigungsflüssigkeit mit einer Temperatur von 15 bis 40°C, vorzugsweise von 20 bis 35°C, insbesondere von 20 bis 30°C, am meisten bevorzugt von 20 bis 25°C, gewaschen. Zweckmäßigerweise wird eine Waschflüssigkeit verwendet, die bezüglich der zu kristallisierenden Acrylsäure bzw. Methacrylsäure eine höhere Reinheit hat als die Mutterlauge aus der Kristallisation. Vorzugsweise enthält die Waschflüssigkeit 90 bis 99,99 Gew.-% Säure, insbesondere 97,0 bis 99,9 Gew.-% Säure, am meisten bevorzugt 99,0 bis 99,8 Gew.-% Säure, jeweils bezogen auf 100 Gew.-% Waschflüssigkeit. Vorteilhafterweise wird als Waschflüssigkeit reine Säure, d.h., ein Reinprodukt mit 99,3 bis 99,8 Gew.-% Acrylsäure bzw. Methacrylsäure, oder die durch Kristallisation aufzureinigende Säurelösung, d.h. der Feed der Kristallisation bzw. die aus der Kondensation des gasförmigen Produktgemischs gemäß Stufe (b) erhaltene Lösung, verwendet. Bei den Verunreinigungen in der Waschflüssigkeit handelt sich meist um Verunreinigungen/Nebenkomponenten aus dem Herstellungs- und/oder Aufreinigungsprozeß der Säure. Die Waschflüssigkeit wird vorzugsweise in einer Menge von 3 bis 80 g Waschflüssigkeit/100 g Kristallisat, insbesondere 5 bis 50 g Waschflüssigkeit/100 g Kristallisat, stärker bevorzugt 10 bis 25 g Waschflüssigkeit/100 g Kristallisat, eingesetzt.

Die Durchführung des Waschvorganges unterliegt keiner Beschränkung. Das Waschen kann einstufig, aber auch mehrstufig durchgeführt werden. Bevorzugt wird mehrstufig gewaschen, insbesondere in zwei Stufen. Das Waschen kann in hierfür üblichen Apparaten kontinuierlich oder diskontinuierlich erfolgen. Vorteilhafterweise werden Zentrifugen, Filternutschen oder Bandfilter verwendet. Zweckmäßigerweise wird bei Einsatz kontinuierlich arbeitender Apparate zur Fest-Flüssig-Trennung, wie Zentrifugen, Dekanter oder Bandfilter, kontinuierlich gewaschen und bei Einsatz diskontinuierlich arbeitender Apparate zur Fest-Flüssig-Trennung, z.B. Filternutschen, diskontinuierlich gewaschen. Das Waschen kann auf Zentrifugen oder Bandfiltern ein- oder mehrstufig durchgeführt werden. Hierbei kann die Waschflüssigkeit auch im Gegenstrom zum Kristallkuchen geführt werden.

Allgemein eignen sich zur Kristallisation der Acrylsäure bzw. Methacrylsäure, aus der dann das erfindungsgemäß zu reinigende Kristallisat erhalten wird, Gemische, die vorzugsweise 65 bis 99,9 Gew.-%, insbesondere 75 bis 99,8 Gew.-%, am meisten bevorzugt 85 bis 99,5 Gew.-% Acrylsäure bzw. Methacrylsäure, jeweils bezogen auf 100 Gew.-% Gemisch, enthalten, wobei der Rest Verunreinigungen/Nebenkomponenten beliebiger Art darstellt, insbesondere Verunreinigungen oder Nebenkomponenten aus dem Herstellungsprozeß der Säure, z.B. der katalytischen Gasphasenoxidation. Vorteilhafterweise wird die Kristallisation als Suspensionskristallisation durchgeführt.

Das verwendete Kristallisationsverfahren unterliegt keiner Beschränkung. Die Kristallisation kann kontinuierlich oder diskontinuierlich, einstufig oder mehrstufig durchgeführt werden. Vorzugsweise erfolgt die Kristallisation einstufig. In einer anderen bevorzugten Ausführungsform der Erfindung wird die Kristallisation als fraktionierte Kristallisation durchgeführt. Üblicherweise werden bei fraktionierter Kristallisation alle Stufen, die ein Kristallisat erzeugen, das reiner ist als die zugeführte Säurelösung, Reinigungsstufen genannt und alle anderen Stufen Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt wird und dieses Kristallisat der jeweiligen Stufe mit dem nächsthöheren Reinheitsgrad zugeführt wird, während der Kristallisationsrückstand der jeweiligen Stufe mit dem nächstniedrigeren Reinheitsgrad zugeführt wird.

Vorteilhafterweise liegt die Temperatur der Lösung während der Kristallisation zwischen 14°C und -30°C, insbesondere zwischen 14°C und -5°C. Der Feststoffgehalt im Kristallisator liegt vorteilhafterweise zwischen 0 und 90 g, bevorzugt zwischen 10 und 80 g Feststoff/100 g Suspension, stärker bevorzugt zwischen 15 und 50 g Feststoff/100 g Suspension.

In einer vorteilhaften Ausgestaltung der Erfindung erfolgt die Kristallisation durch Kühlung von Apparatewänden oder durch Verdampfung der Lösung im Vakuum. Bei der Kristallisation durch Kühlung von Apparatewänden wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere bevorzugte Ausführungsform bei der Kühlungskristallisation ist die Verwendung von Kühlscheibenkristallem, wie sie z. B. von der Firma Gouda (Holland) hergestellt werden. Bei einer weiteren Variante zur Kristallisation durch Kühlung wird die Wärme über herkömmliche Wärmeüberträger (bevorzugt Rohrbündel- oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche Verdampfung der Lösung im Vakuum erfolgen.

Zweckmäßigerweise werden nach der Kristallisation die erhaltenen Säurekristalle von der Mutterlauge abgetrennt. Die Art der Durchführung der Abtrennung unterliegt keinen besonderen Beschränkungen. Es eignen sich alle bekannten Verfahren der Fest-Flüssig-Trennung. In einer Ausführungsform werden die Kristalle durch Filtrieren und/oder Zentrifugieren von der Mutterlauge abgetrennt. Vorteilhafterweise wird dem Filtrieren oder Zentrifugieren eine Voreindickung der Suspension, z.B. durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen. Es besteht auch die Möglichkeit, daß zusätzlich zu dem erfindungsgemäßen Waschprozeß ein Schwitzen der Kristalle oder des Kristallkuchens vorgesehen wird. Hierbei handelt es sich um ein lokales Abschmelzen verunreinigter Bereiche. Bevorzugt erfolgt die Durchführung des Schwitzens auf Zentrifugen oder Bandfiltern.

Das erfindungsgemäße Reinigungsverfahren wird vorteilhafterweise mit Kristallisaten durchgeführt, die 85 bis 99,9 Gew.-% Säure, insbesondere 95 bis 99,5 Gew.-% Säure, jeweils bezogen auf 100 Gew.-% Kristallisat, enthalten. An Verunreinigungen oder Nebenkomponenten enthalten die Kristallisate hauptsächlich solche, die aus der Herstellung und/oder Aufreinigung der Rohsäure stammen. Die nach Durchführung des erfindungsgemäßen Reinigungsverfahrens erhaltenen Säurekristalle enthalten in der Regel 95 bis 99,9 Gew.-% Säure, insbesondere 99,0 bis 99,8 Gew.-% Säure, stärker bevorzugt 99,3 bis 99,8 Gew.-% Säure, jeweils bezogen auf 100 Gew.-% Säurekristalle.

Das erfindungsgemäße Reinigungsverfahren ermöglicht auf einfache Art und Weise durch Waschen des Kristallisats eine signifikante Erhöhung der Reinheit um 20 bis 70 % je nach Verunreinigungskomponente, bezogen auf das ungewaschene Kristallisat.

In einer Ausgestaltung betrifft die Erfindung ein Verfahren zur Herstellung von Acrylsäure oder Methacrylsäure, das die oben definierten Stufen (a) bis (d) umfaßt.

### Stufe (a):

In Stufe (a) wird ein gasförmiges Produktgemisch hergestellt, das im wesentlichen die Zusammensetzung eines Reaktionsgemischs der katalytischen Gasphasenoxidation von C₃- bzw. C₄-Alkanen, -Alkenen, -Alkanolen und/oder -Alkanalen und/oder Vorstufen davon zu Acrylsäure oder Methacrylsäure hat. Besonders vorteilhaft wird das gasförmige Produktgemisch durch katalytische Gasphasenoxidation von Propen, Acrolein, tert.-Butanol, Isobuten, Isobutan, Isobutyraldehyd, Methacrolein, Isobuttersäure oder Methyl-tert.-butylether hergestellt. Als Ausgangsverbindungen können alle Vorstufen der genannten Verbindungen verwendet werden, bei denen sich die eigentliche C₃-/C₄-Ausgangsverbindung erst intermediär während der Gasphasenoxidation bildet. Beispielhaft genannt für die Herstellung von Methacrylsäure sei Methyl-tert.-butylether oder Isobuttersäure.

Besonders vorteilhaft ist die katalytische Gasphasenreaktion von Propen und/oder Acrolein zu Acrylsäure mit molekularem Sauerstoff nach bekannten Verfahren, insbesondere wie sie in den Druckschriften DE-A-1 962 431, DE-A-2 943 707, DE-C-1 205 502, DE-A-195 08 558, EP-A-0 257 565, EP-A-0 253 409, DE-A-2 251 364, EP-A-0 117 146, GB-B-1 450 986 und EP-A-0 293 224 beschrieben sind. Vorzugsweise wird hierbei bei Temperaturen zwischen 200 und 450°C und gegebenenfalls erhöhtem Druck gearbeitet. Vorzugsweise werden als heterogene Katalysatoren oxidische Mehrkomponenten-Katalysatoren auf der Basis der Oxide von Molybdän, Bismut und Eisen in der 1. Stufe (Oxidation von Propen zu Acrolein) und der Oxide von Molybdän und Vanadium in der 2. Stufe (Oxidation von Acrolein zu Acrylsäure) eingesetzt. Wird Propan als Ausgangsstoff verwendet, so kann dieses zu einem Propen-/Propan-Gemisch umgesetzt werden durch: katalytische Oxidehydrierung, wie z. B. in Catalysis Today 24 (1995), 307 bis 313 oder US-A 5,510,558 beschrieben; durch homogene Oxidehydrierung, wie z. B. in CN-A 1 105 352 beschrieben; oder durch katalytische Dehydrierung, wie z. B. in EP-A 0 253 409, EP-A 0 293 224, DE-A 195 08 558 oder EP-A 0 117 146 beschrieben. Bei Einsatz eines Propen-/Propan-Gemischs wirkt Propan als Verdünnungsgas. Geeignete Propen-/Propan-Gemische sind auch Raffineriepropen (70% Propen und 30% Propan) oder Crackerpropen (95% Propen und 5% Propan). Grundsätzlich können Propen-/Propan-Gemische wie die oben genannten mit Sauerstoff oder Luft oder einem Gemisch aus Sauerstoff und Stickstoff jeder Zusammensetzung zu Acrolein und Acrylsäure oxidiert werden.

Die Umsetzung von Propen zu Acrylsäure ist stark exotherm. Das Reaktionsgas, das neben den Edukten und Produkten vorteilhafterweise ein inertes Verdünnungsgas, z. B. Luftstickstoff, einen oder mehrere gesättigte C₁-C₆-Kohlenwasserstoffe, insbesondere Methan und/oder Propan und/oder Wasserdampf enthält, kann daher nur einen kleinen Teil der Reaktionswärme aufnehmen. Obwohl die Art der verwendeten Reaktoren an sich keiner Beschränkung unterliegt, werden meist Rohrbündelwärmetauscher verwendet, die mit dem Oxidationskatalysator gefüllt sind, da bei diesen der überwiegende Teil der bei der Reaktion freiwerdenden Wärme durch Konvektion und Strahlung an die gekühlten Rohrwände abgeführt werden kann.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure, Formaldehyd, weitere Aldehyde und Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% Maleinsäureanhydrid und 20 bis 98 Gew.-%, vorzugsweise 50 bis 98 Gew.-%. inerte Verdünnungsgase. Als inerte Verdünnungsgase sind insbesondere gesättigte C₁-C₆-Kohlenwasserstoffe, wie 0 bis 90 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Verdünnungsgas, enthalten.

Die Methacrylsäure kann analog zu Acrylsäure durch katalytische Gasphasenreaktion von C₄-Ausgangsverbindungen mit molekularem Sauerstoff hergestellt werden. Besonders vorteilhaft ist die Methacrylsäure, z. B. durch katalytische Gasphasenoxidation von Isobuten, Isobutan, tert.-Butanol, Isobutyraldehyd, Methacrolein oder Methyl-tert.-butylether erhältlich. Als Katalysatoren werden ebenfalls übergangsmetallische Mischoxidkatalysatoren (z. B, Mo, V, W und/oder Fe) verwendet. Besonders geeignete Verfahren sind solche, bei denen die Herstellung ausgehend von Methacrolein erfolgt, insbesondere dann, wenn das Methacrolein durch gasphasenkatalytische Oxidation von tert.-Butanol, Isobutan oder Isobuten oder durch Umsetzung von Formaldehyd mit Propionaldehyd gemäß EP-B 0 092 097 oder EP-B 0 058 927 erzeugt wird. Somit besteht auch die Möglichkeit, Methacrylsäure zweistufig herzustellen durch (1) Kondensation von Propionaldehyd mit Formaldehyd (in Gegenwart eines sekundären Amins als Katalysator) zu Methacrolein und (2) anschließende Oxidation des Methacroleins zu Methacrylsäure.

Ebenso wie bei der Herstellung der Acrylsäure wird nicht reine Methacrylsäure, sondern ein gasförmiges Gemisch erhalten, das neben der Methacrylsäure als Nebenkomponenten im wesentlichen nicht umgesetztes Methacrolein und/oder Wasserdampf, Kohlenmonoxid, Kohlendioxid, Stickstoff, Sauerstoff, Essigsäure, Propionsäure, weitere Aldehyde und Maleinsäureanhydrid enthalten kann. Das erfindungsgemäße Verfahren wird insbesondere dann eingesetzt, wenn das Reaktionsgemisch, 0,02 bis 2 Gew.-% Methacrolein bezogen auf das gesamte Reaktionsgemisch und ansonsten im wesentlichen die gleichen entsprechenden Bestandteile wie bei der Herstellung der Acrylsäure enthält.

### Stufe (b):

In Stufe (b) wird das in Stufe (a) erhaltene Reaktionsprodukt einer Kondensation, insbesondere einer Partial- oder Totalkondensation, unterzogen, wobei eine Lösung erhalten wird. Die Kondensation kann nach üblichen Verfahren ein- oder mehrstufig erfolgen, und die Art der Kondensation unterliegt dabei keiner besonderen Beschränkung. Vorteilhafterweise wird die Kondensation mit einem Direktkondensator durchgeführt, wobei bereits erzeugtes Kondensat mit dem heißen gasförmigen Reaktionsprodukt in Kontakt gebracht wird. Als Apparate für die Kondensation eignen sich insbesondere Sprühwäscher, Venturiwäscher, Blasensäulen oder Apparate mit berieselten Oberflächen.

Die durch Partial- oder Totalkondensation des Reaktionsproduktes aus Stufe (a) erhaltene Mischung enthält vorzugsweise 65 bis 99,5 Gew.-% Acrylsäure bzw. Methacrylsäure, 0,1 bis 35 Gew.-% Wasser, daneben 0,1 bis 15 Gew.-% Verunreinigungen, insbesondere, jeweils bezogen auf 100 Gew.-% Kondensat, 0,01 bis 5 Gew.-% Acrolein bzw. Methacrolein, 0,05 bis 5 Gew.-% Essigsäure, 0,01 bis 5 Gew.-% Propionsäure, 0,01 bis 5 Gew.-% Formaldehyd, 0,01 bis 5 Gew.-% weitere Aldehyde und 0,01 bis 5 Gew.-% Maleinsäure. Besonders bevorzugt wird bei der Kondensation ein Gemisch erhalten, welches 85 bis 99,5 Gew.-%, insbesondere 90 bis 98,5 Gew.-%, Acrylsäure bzw. Methacrylsäure, 0,1 bis 15 Gew.-%, insbesondere 0,5 bis 10 Gew.-%, Wasser, daneben 0,5 bis 5 Gew.-% Verunreinigungen, insbesondere, jeweils bezogen auf 100 Gew.-% Kondensat, 0,01 bis 3 Gew.-% Acrolein bzw. Methacrolein, 0,1 bis 3 Gew.-% Essigsäure, 0,01 bis 3 Gew.-% Propionsäure 0,01 bis 3 Gew.-% Formaldehyd, 0,01 bis 3 Gew.-% weitere Aldehyde und 0,01 bis 3 Gew.-% Maleinsäure enthält.

### Stufe (c):

In Stufe (c) wird die in Stufe (b) erhaltene Lösung, die Acrylsäure bzw. Methacrylsäure enthält, kristallisiert. Somit wird die in der Kondensationsstufe erhaltene Lösung direkt der Kristallisation zugeführt. Hierbei wird ohne Zusatz eines Lösungsmittels, insbesondere ohne Zusatz eines organischen Lösungsmittels, gearbeitet.

Die Kristallisation wird vorzugsweise durchgeführt wie vorstehend beschrieben, wobei das Kristallisat und die Mutterlauge erhalten werden.

### Stufe (d):

In Stufe (d) wird das Kristallisat der vorhergehenden Stufe nach Abtrennung von der Mutterlauge, die wie oben beschrieben durchgeführt wird, gemäß dem erfindungsgemäßen Reinigungsverfahren gewaschen, um die aufgereinigte Acrylsäure bzw. Methacrylsäure zu erhalten.

Somit stellt die vorliegende Erfindung ein Verfahren zur Herstellung und Reinigung von Acrylsäure oder Methacrylsäure zur Verfügung, bei dem durch ein einfaches Reinigungsverfahren eine signifikante Erhöhung der Reinheit der gewünschten Säure erreicht wird.

Die Erfindung wird anhand des folgenden Beispiels, das eine bevorzugte Ausfilhrungsform der Erfindung darstellt, näher erläutert.

### Beispiel

Eine Roh-Acrylsäure mit der in Tabelle 1 unten angegebenen Zusammensetzung wurde durch Kühlungskristallisation in einem Kühlscheibenkristallisator (100 l) kontinuierlich bei einer Temperatur von 9,4 °C kristallisiert. Der Feststoffgehalt im Kristallisator betrug 28 g Feststoff/ 100 g Suspension.

**Tabelle 1:**

| **Zusammensetzung der zu kristallisierenden Roh-Acrylsäure:** | | |
|---|---|---|
| **Acrylsäure** | 96,43 | Gew.-% |
| **Essigsäure** | 0,84 | Gew.-% |
| **Propionsäure** | 610 | ppm |
| **Furan(II)aldehyd** | 0,41 | Gew.-% |
| **Benzaldehyd** | 240 | ppm |
| **Maleinsäure** | 200 | ppm |
| **Allylacrylat** | 0,22 | Gew.-% |
| **Wasser** | 1,43 | Gew.-% |
| **Sonstige** | 0,565 | Gew.-% |

Nach der Kristallisation erfolgte die Abtrennung der Acrylsäure von der Mutterlauge auf einer zweistufigen Schubzentrifuge (Durchmesser 400 mm, Drehzahl 1900 l/min) mit Gegenstromwäsche. Die Menge an Waschflüssigkeit lag bei 25 g Waschflüssigkeit / 100 g Kristallkuchen. Die Zusammensetzung der Waschflüssigkeit war wie folgt:

**Tabelle 2:**

| **Zusammensetzung der Waschflüssigkeit** | | |
|---|---|---|
| **Acrylsäure** | 99,67 | Gew.-% |
| **Essigsäure** | 1333 | ppm |
| **Propionsäure** | 342 | ppm |
| **Furan(II)aldehyd** | 263 | ppm |
| **Benzaldehyd** | 85 | ppm |
| **Maleinsäure** | 124 | ppm |
| **Allylacrylat** | 54 | ppm |
| **Wasser** | 540 | ppm |
| **Sonstige** | 587 | ppm |

In Abhängigkeit von der Temperatur der Waschflüssigkeit ergab sich die in Tabelle 3 angegebene Zusammensetzung des Kristallisats. Hierbei sind alle Angaben, außer für Acrylsäure, auf ppm bezogen, während die Menge an Acrylsäure auf Gew.-% bezogen ist.

**Tabelle 3:**

| **Zusammensetzung des Kristallisats als Funktion der Temperatur der Waschflüssigkeit** | | | | | |
|---|---|---|---|---|---|
| | **Ohne Wäsche** | **15°C** | **20°C** | **25 °C** | **30 °C** |
| **Acrylsäure** | 99,62 | 99,75 | 99,76 | 99,77 | 99,77 |
| **Essigsäure** | 1920 | 1504 | 1436 | 1422 | 1386 |
| **Propionsäure** | 219 | 189 | 189 | 182 | 174 |
| **Furan(II)aldehyd** | 281 | 139 | 118 | 94 | 94 |
| **Benzaldehyd** | 19 | 9 | 7 | <5 | <5 |
| **Maleinsäure** | 18 | 7 | 6 | <5 | <5 |
| **Allylacrylat** | 165 | 79 | 68 | 53 | 51 |
| **Wasser** | 510 | 261 | 248 | 241 | 241 |
| **Sonstige** | 624 | 301 | 294 | 270 | 254 |

Somit führt das erfindungsgemäße Waschen des Kristallkuchens zu einer signifikanten Erhöhung der Reinheit der Acrylsäure.

## Patentansprüche

1. Verfahren zur Reinigung von Acrylsäure oder Methacrylsäure durch Kristallisation unter Erhalt eines Kristallisats und einer Mutterlauge, **dadurch gekennzeichnet, daß** das Kristallisat mit einer Acrylsäure bzw. Methacrylsäure enthaltenden Waschflüssigkeit mit einer Temperatur von 15 bis 40° C gewaschen wird.

2. Verfahren' nach Anspruch 1, **dadurch gekennzeichnet, daß** eine Waschflüssigkeit verwendet wird, die bezüglich der Acrylsäure bzw. Methacrylsäure eine höhere Reinheit als die Mutterlauge hat.

3. Verfahren nach Anspruch 1 oder 2, **dadurch gekennzeichnet, daß** eine Waschflüssigkeit mit 97,0 bis 99,9 Gew.-% Acrylsäure bzw. Methacrylsäure, bezogen auf 100 Gew.-% Waschflüssigkeit, verwendet wird.

4. Verfahren nach einem der vorhergehenden Ansprüche, **dadurch gekennzeichnet, daß** die Kristallisation als Suspensionskristallisation durchgeführt wird.

5. Verfahren nach einem der vorhergehenden Ansprüche, **dadurch gekennzeichnet, daß** eine Waschflüssigkeit mit einer Temperatur von 20 bis 35° C verwendet wird.

6. Verfahren nach einem der vorhergehenden Ansprüche, **dadurch gekennzeichnet, daß** eine Waschflüssigkeit mit einer Temperatur von 20 bis 25°C verwendet wird.

7. Verfahren nach einem der vorhergehenden Ansprüche, **dadurch gekennzeichnet, daß** mit einer Menge von 3 bis 80 g Waschflüssigkeit/100g Kristallisat gewaschen wird.

8. Verfahren nach einem der vorhergehenden Ansprüche, **dadurch gekennzeichnet, daß** als Waschflüssigkeit ein Reinprodukt mit 99,3 bis 99,8 Gew.-% Acrylsäure bzw. Methacrylsäure verwendet wird.

9. Verfahren nach einem der vorhergehenden Ansprüche, **dadurch gekennzeichnet, daß** das Waschen mehrstufig durchgeführt wird.

10. Verfahren zur Herstellung von Acrylsäure oder Methacrylsäure durch
(a) Herstellung eines gasförmigen Produktgemisches, das im wesentlichen die Zusammensetzung eines Reaktionsgemisches der katalytischen Gasphasenoxidation von C₃- bzw. C₄-Alkanen, -Alkenen, -Alkanolen und/oder -Alkanalen und/oder Vorstufen davon zu Acrylsäure oder Methacrylsäure hat,
(b) Kondensation des gasförmigen Produktgemisches,
(c) Kristallisation der Acrylsäure oder Methacrylsäure aus der in Stufe (b) erhaltenen Lösung und
(d) Reinigung der Acrylsäure bzw. Methacrylsäure aus dem Kristallisat der vorhergehenden Stufe nach einem Verfahren wie in einem der vorhergehenden Ansprüche definiert.

## Claims

1. A process for the purification of acrylic acid or methacrylic acid by crystallization to obtain crystals and a mother liquor, wherein the crystals are washed with a wash liquid containing acrylic acid or methacrylic acid and having a temperature from 15 to 40°C.

2. A process as claimed in claim 1, wherein a wash liquid which has a higher purity than the mother liquor with respect to acrylic acid or methacrylic acid is used.

3. A process as claimed in claim 1 or 2, wherein a wash liquid comprising from 97.0 to 99.9% by weight, based on 100% by weight of wash liquid, of acrylic acid or methacrylic acid is used.

4. A process as claimed in any of the preceding claims, wherein the crystallization is carried out as a suspension crystallization.

5. A process as claimed in any of the preceding claims, wherein a wash liquid having a temperature of from 20 to 35°C is used.

6. A process as claimed in any of the preceding claims, wherein a wash liquid having a temperature of from 20 to 25°C is used.

7. A process as claimed in any of the preceding claims, wherein washing is effected with an amount of from 3 to 80 g of wash liquid/100 g of crystals.

8. A process as claimed in any of the preceding claims, wherein the wash liquid used is a pure product comprising from 99.3 to 99.8% by weight of acrylic acid or methacrylic acid.

9. A process as claimed in any of the preceding claims, wherein the washing is carried out in a plurality of stages.

10. A process for the preparation of acrylic acid or methacrylic acid by
(a) preparation of a gaseous product mixture which essentially has the composition of a reaction mixture of the catalytic gas-phase oxidation of C₃- or C₄-alkanes, C₃- or C₄-alkenes, C₃- or C₄-alkanols and/or C₃- or C₄-alkanals and/or intermediates thereof to acrylic acid or methacrylic acid,
(b) condensation of the gaseous product mixture,
(c) crystallization of the acrylic acid or methacrylic acid from the solution obtained in stage (b) and
(d) purification of the acrylic acid or methacrylic acid from the crystals of the preceding stage by a process as defined in any of the preceding claims.

## Revendications

1. Procédé de purification d'acide acrylique ou d'acide méthacrylique par cristallisation avec obtention d'un produit de cristallisation et d'une lessive-mère, **caractérisé en ce que** le produit de cristallisation est lavé avec un liquide de lavage contenant de l'acide acrylique ou respectivement de l'acide méthacrylique et présentant une température de 15 à 40°C.

2. Procédé suivant la revendication 1, **caractérisé en ce qu'**on utilise un liquide de lavage qui, par rapport à l'acide acrylique ou respectivement l'acide méthacrylique, présente une pureté supérieure à la lessive-mère.

3. Procédé suivant l'une des revendications 1 et 2, **caractérisé en ce qu'**on utilise un liquide de lavage comportant 97,0 à 99,9 % en poids d'acide acrylique ou d'acide méthacrylique, par rapport à 100 % en poids de liquide de lavage.

4. Procédé suivant l'une des revendications précédentes, **caractérisé en ce que** la cristallisation est effectuée sous la forme d'une cristallisation en suspension.

5. Procédé suivant l'une des revendications précédentes, **caractérisé en ce qu'**on utilise un liquide de lavage ayant une température de 20 à 35°C.

6. Procédé suivant l'une des revendications précédentes, **caractérisé en ce qu'**on utilise un liquide de lavage ayant une température de 20 à 25°C.

7. Procédé suivant l'une des revendications précédentes, **caractérisé en ce qu'**on lave avec une quantité de 3 à 80 g de liquide de lavage/100 g de produit de cristallisation.

8. Procédé suivant l'une des revendications précédentes, **caractérisé en ce que**, comme liquide de lavage, on utilise un produit pur ayant 99,3 à 99,8 % en poids d'acide acrylique ou d'acide méthacrylique.

9. Procédé suivant l'une des revendications précédentes, **caractérisé en ce que** le lavage est effectué en plusieurs étapes.

10. Procédé de préparation d'acide acrylique ou d'acide méthacrylique par
(a) préparation d'un mélange gazeux qui présente essentiellement la composition d'un mélange réactionnel de l'oxydation catalytique en phase gazeuse d'alcanes, alcènes, alcanols et/ou alcanals en C₃-C₄ et/ou de leurs précurseurs en acide acrylique ou acide méthacrylique,
(b) condensation du mélange gazeux,
(c) cristallisation de l'acide acrylique ou de l'acide méthacrylique à partir de la solution obtenue dans l'étape (f),
(d) purification de l'acide acrylique ou de l'acide méthacrylique à partir du produit de cristallisation de l'étape précédente, suivant un procédé tel que défini dans l'une des revendications précédentes.
